# EUROPEAN PATENT SPECIFICATION

(11) **EP 2 437 658 B1**
(45) Date of publication and mention of the grant of the patent: **21.08.2019**
(21) Application number: 09779625.4
(22) Date of filing: 03.06.2009
(51) Int. Cl.: A61B 5/0476

(54) **METHOD FOR ASSESSING THE SUSCEPTIBILITY OF A HUMAN INDIVIDUAL SUFFERING FROM A PSYCHIATRIC OR NEUROLOGICAL DISORDER TO NEUROMODULATION TREATMENT**
VERFAHREN ZUR BEURTEILUNG DES ANSPRECHENS EINER PERSON MIT EINER PSYCHIATRISCHEN ODER NEUROLOGISCHEN ERKRANKUNG AUF EINE NEUROMODULATORISCHE BEHANDLUNG
PROCÉDÉ POUR ÉVALUER LA SUSCEPTIBILITÉ D'UN ÊTRE HUMAIN SOUFFRANT DE TROUBLES PSYCHIATRIQUES OU NEUROLOGIQUES À UN TRAITEMENT DE NEUROMODULATION

(43) Date of publication of application: 11.04.2012
(73) Proprietor: MYnd Analytics, Inc., Mission Viejo, CA 92691 (US)
(72) Inventor: ARNS, Martijn Wilco, NL-6573 DZ Beek Ubbergen (NL); SPRONK, Desirée Bianca, NL-6541 NT Nijmegen (NL)
(74) Representative: Hargreaves, Timothy Edward
(86) International application number: PCT/EP2009/056812
(87) International publication number: WO 2010/139361

(56) References cited:
- WO-A1-01/58351
- JOHNSTONE J ET AL: "Clinical database development: characterization of EEG phenotypes." CLINICAL EEG AND NEUROSCIENCE : OFFICIAL JOURNAL OF THE EEG AND CLINICAL NEUROSCIENCE SOCIETY (ENCS) APR 2005, vol. 36, no. 2, April 2005 (2005-04), pages 99-107, XP008118395 ISSN: 1550-0594
- YANAI ICHIRO ET AL: "Changes in auditory P300 in patients with major depression and silent cerebral infarction" JOURNAL OF AFFECTIVE DISORDERS, vol. 46, no. 3, December 1997 (1997-12), pages 263-271, XP002567568 ISSN: 0165-0327
- SPRONK DESIRÉE ET AL: "Long-term effects of left frontal rTMS on EEG and ERPs in patients with depression." CLINICAL EEG AND NEUROSCIENCE : OFFICIAL JOURNAL OF THE EEG AND CLINICAL NEUROSCIENCE SOCIETY (ENCS) JUL 2008, vol. 39, no. 3, July 2008 (2008-07), pages 118-124, XP008118357 ISSN: 1550-0594

## Description

The invention relates to a method for assessing the susceptibility of a human individual suffering from a psychiatric or neurological disorder to neuromodulation treatment. The invention furthermore relates to a device for use in such a method.

The treatment of psychiatric and neurological complaints is constantly subject to new insights and scientific developments. Nowadays medication and psychotherapy are applied on a large scale. Over the past few years there has also been a clear shift from a systemic - or drug treatment - to a more local treatment which is more directed to a specific area or network in the brain. These techniques are also often called Neuromodulation techniques. Examples of local neuromodulation methods are the application of repetitive Transcranial Magnetic Stimulation (rTMS or TMS), transcranial Direct Current Stimulation (tDCS), deep-brain stimulation (DBS) and EEG-biofeedback or neurofeedback. Another development, which is complementary to the above mentioned, is Personalized Medicine. Its goal is to personalize therapy on the basis of genotypic and phenotypic information in order to reach higher effectiveness for different kinds of treatments.

These new - more local - treatment options provide us with a completely new view of treatment of brain disorders whereby broad categories such as "depression" and "schizophrenia" are no longer relevant. These new treatment methods rather focus on a specific complaint (such as auditory hallucinations, depressed mood or tinnitus) rather than blindly applying the same treatment to all patients. The most critical factor of these new treatment methods is the personalizing of these treatments. The development of these new local treatment methods is therefore complimentary to the development of personalized medicine. Personalized medicine is a new approach with the goal to create more effective treatments through personalizing treatment.

Drug treatments in psychiatry only have limited effectiveness (40-60% for depression (Keller et al., 2000) and 60-80% for ADHD (Swanson et al., 1993). Considering this low to moderate effectiveness there seems to be a need for a personalized approach in treating psychiatric and neurological disorders.

JOHNSTONE J ET AL: "Clinical database development: characterization of EEG phenotypes.", CLINICAL EEG AND NEUROSCIENCE : OFFICIAL JOURNAL OF THE EEG AND CLINICAL NEUROSCIENCE SOCIETY (ENCS) APR 2005, calls for statistical analysis methods to be applied to a broad clinical database of individuals diagnosed with neurobehavioral disorders in order to empirically define clusters of individuals who may be responsive to specific neurophysiologically based treatment Interventions, namely administration of psychoactive medication and/or EEG neurofeedback.

It is an objective of the invention, among others, to provide a reliable, easy and/or efficient method to assess the susceptibility of a human individual to neuromodulation treatment.

In order to accomplish that objective, a method is provided for assessing the susceptibility of a human individual suffering from a psychiatric or neurological disorder, in particular with depressed mood as a predominant feature, to neuromodulation treatment, in particular repetitive Transcranial Magnetic Stimulation (rTMS), the method comprising:
a) Providing a dataset comprising electroencephalographic (EEG) activity and Event Related Potentials (ERP) data of said human individual, and;
b) Assessing the susceptibility of said human individual to neuromodulation treatment based on said dataset.

The invention is based on the recognition that brain imaging data (e.g. QEEG, ERPs, MEG, fMRI, PET scans) can be considered as a phenotype which includes both the effects of nature and nurture. Therefore it can give a reliable indication of the 'state of the system'. This potentially makes data obtained from neuroimaging a reliable predictor for treatment outcome for treatments such as rTMS and medication (e.g. antidepressants or stimulants).

The brain function assessment according to the invention concern electroencephalographic (EEG) activity and EEG related measures (Event Related Potentials). EEG is the electrical activity from the brain and is a widely used technique to measure brain function both for research and clinical uses'. Event Related Potentials are potentials which are present in the EEG when a subject is presented with a task, where for instance auditory stimuli are presented to the subject. By averaging the EEG at exactly the presentation of these stimuli an Evoked Potential (EP) or Event Related Potential (ERP) is obtained.

Often such tasks employ different stimuli, for example in an Oddball task a frequent and infrequent tone is presented and the subject is instructed to respond to the infrequent tones, by pressing a button. The averaged EEG activity of the 'infrequent' tone is then the Oddball ERP and the averaged EEG activity to the 'frequent' tone is then the Oddball Background ERP. Where ERP is mentioned in this patent we refer to any Event Related Potential and not limited to the Oddball ERP.

In the preferred embodiment of this invention these EEG and ERP data are recorded and collected using the standardized Brain Resource Company methods and equipment. More information on these methods has also been published in the scientific literature (Gordon, 2003). To summarize, EEG data are collected from 26 scalp locations but include at least the following standard EEG locations: F3, Fz, F4, Cz and Pz. The data are at least recorded under the following conditions: a) Two minutes Eyes Open; b) Two minutes Eyes Closed and c) During an auditory oddball paradigm with an infrequent high-pitched tone (50 ms, 75 dB tone at 1000 Hz; total 280 stimuli, quasi random) and a frequent low-pitched tone (50 ms 75 dB tone at 500 Hz; total 60 stimuli, quasi random) with inter stimulus interval of 1 second. The minimum embodiment consists of a single channel of EEG and related ERP's, therefore the above does not imply multiple channels are required for this patent and single-channel recordings are explicitly also covered.

It should be noted that although the use of EEG is described, the disclosure also relates to the use of Magneto encephalography (MEG) data, since the MEG technique yields similar results as with EEG.

This invention applies to patients with neurological, psychological and/or psychiatric complaints in the broadest sense. In the preferred embodiment the invention applies to patients with a complaint of depressive mood (including but not limited to Major Depressive Disorder, bipolar disorder, Dysthymia, Mood Disorders or depressive mood as a comorbid psychiatric complain in for example Tinnitus or Parkinson). Below EEG and ERP predictors of favourable and unfavourable treatment response are outlined. In the broadest sense this applies to all neuromodulation treatments (rTMS, TMS, tDCS)at any scalp location, but in the preferred embodiment this specifically applies to rTMS therapy (magnetic brain stimulation or repetitive Transcranial Magnetic Stimulation) applied at any frequency to the right or left frontal cortex. The predictors for non-response implicate those people will not clinically benefit from neuromodulation treatment and the predictors for response implicate those people will respond to neuromodulation treatment.

According to the invention step b of assessing the susceptibility comprises:
- Comparing said dataset with reference data comprising EEG and ERP data from a control group, and;
- Assessing the susceptibility of said human individual to neuromodulation treatment based on said comparison.

The provided dataset is compared with reference data for assessing the susceptibility. It is however also possible to compare the provided dataset with predetermined absolute values.

According to the invention the susceptibility is assessed as negative in step b if the data in said dataset meets any one of the conditions chosen from the first group of: a P300 ERP amplitude that is increased compared to the reference data; an EEG amplitude in the theta and/or the delta frequency range that is increased compared to the reference data; an alpha peak frequency that is lower than 10Hz.

Tests have indicated that these conditions provide reliable indications for the susceptibility of neuromodulation treatment. As described above, the data for the various conditions can be compared to reference data. It is however also possible to establish the conditions based on absolute values of the various parameters in the conditions.

According to a further preferred embodiment said increased P300 ERP amplitude comprises a P300 Oddball amplitude. More preferably, the amplitude is considered as increased if the amplitude is higher than the average amplitude. More preferably the P300 ERP amplitude is larger than 15 µV and even more preferably larger than 20 µV. Furthermore, the dataset comprises EEG data measured at the Pz location.

According to a further preferred embodiment the increased amplitude of slow EEG exceeds 70 µV, preferably 80 µV and more preferably 90 µV. Preferably the dataset comprises EEG data of the human individual measured with his eyes closed, preferably measured at the Pz location. It should be noted that with the delta frequency band frequencies are meant in a range of 1,5 - 3,5 Hz, while the theta frequency band comprises frequencies in the range of 4 - 7,5 Hz.

According to the invention the alpha peak frequency is lower than 10 Hz, preferably lower than 9 Hz and more preferably lower than 8 Hz. The alpha peak frequency comprises the maximum amplitude in the 4 - 14 Hz frequency band. Preferably the data comprises EEG data of the human individual measured with his eyes closed and preferably measured at the Fz location.

According to a further preferred embodiment, the susceptibility is assessed as positive in step b if none of the conditions of the first group are met and if the data in said dataset meets any one of the conditions chosen from a second group of: a frontal EEG amplitude in the alpha and/or beta frequency range that is increased compared to the reference data; an EEG amplitude in the beta frequency range that is increased compared to the reference data.

The beta frequency band comprises the frequency range of 14,5 - 30 Hz.

According to a further preferred embodiment the increased frontal fast EEG amplitude is higher than 30 µV, preferably higher than 35 µV and more preferably higher than 40 µV. Preferably the data of fast EEG comprises data of the human individual measured with his eyes closed. And preferably the data of the frontal fast EEG comprises data measured from the F3, Fz and/or F4 locations.

According to a further preferred embodiment the data of the beta EEG comprises data measured from the Pz location, wherein the increased beta EEG amplitude is higher than 20 µV, more preferably higher than 25 µV. It is also possible that the data of the beta EEG comprises data measured from the Cz location, wherein the increased beta EEG amplitude is higher
than 25 µV, more preferably higher than 30 µV. Furthermore, the data comprising beta EEG can comprise data measured from the Fz location, wherein the increased beta EEG amplitude is higher than 15 µV, more preferably higher than 20 µV.

Preferably the data of beta EEG comprises data of the human individual measured with his eyes closed.

According to a further preferred embodiment the susceptibility is assessed as negative if the EEG data of the human individual comprises paroxysmal, neurological or epileptic EEG data.

The invention furthermore relates to a device for use in the assessment of the susceptibility of a human individual suffering from a psychiatric or neurological disorder to neuromodulation treatment according to the invention, wherein the device comprises input means for inputting a dataset comprising EEG and ERP data from the human individual and processing means configured to execute the disclosed method for assessing the susceptibility based on said dataset and output means for outputting said assessment based on the result from the processing means.

The invention will be further elucidated using the following example and figures, wherein:
- Figure 1 shows an example of an individual ERP compared to a control group, and;
- Figure 2 shows the results of the method according to the invention.

According to the invention, the following selection method comprising steps 1 - 3 for assessing the susceptibility to rTMS treatment is used:

In the whole process predictors for non-response are most important and have priority over predictors for a good response. Hence, any predictor for non-response invalidates predictors for response.

Step 1) A-priori predictors for non-response are: Paroxysmal, neurological or epileptic EEG. This occurs in 3-5% of patients, even without the symptoms of epilepsy or other neurological problems. These patients need neurological follow up: This group will possibly respond to anticonvulsant medication or SMR Neurofeedback.

Step 2) Prediction of Non-response to Neuromodulation based on pre-treatment EEG and ERP parameters, any of the following observations indicates that someone will be a non-responder to neuromodulation treatment:
- Increased P300 ERP amplitude (higher than average amplitude). This is a P300 oddball amplitude at Pz of more than 20 µV (microvolt), or;
- Increased slow EEG (Delta and/or Theta EEG ampltude) . This is the EEG amplitude in the Delta frequency band (1,5- 3,5 Hz) + EEG amplitude in the Theta frequency band (4-7,5 Hz) during Eyes Closed exceeding 90 µV at location Pz, or;
- A slowed alpha peak frequency (the maximum amplitude in the 4-14 Hz frequency band). This is the alpha peak frequency measured at Fz during eyes closed slower than 8 Hz.

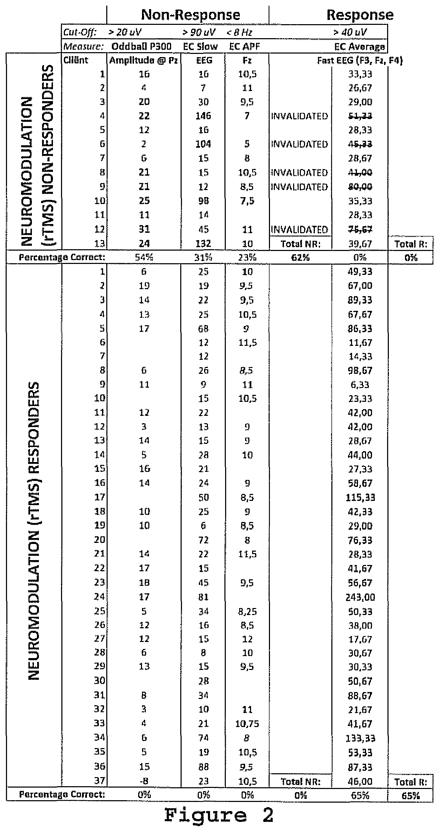

An analysis based on 50 patients treated with rTMS showed that combining these 3 measures has resulted in a 62% correct identification of Non-Responders to rTMS treatment, with no false positive findings, also see figure 2.

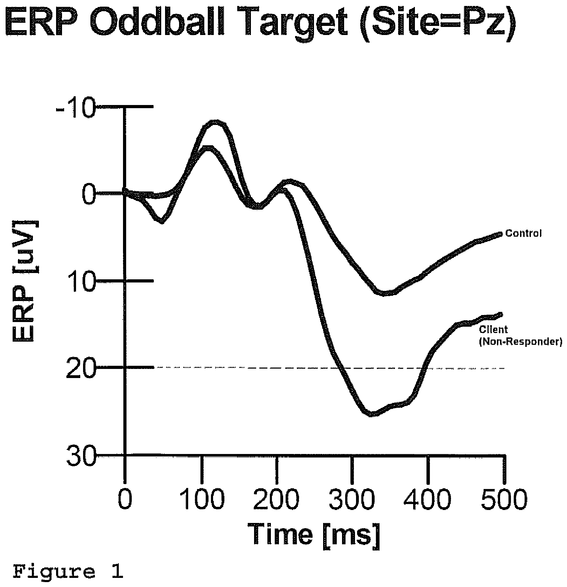

Figure 1 shows an example of individual data predicting a negative treatment-outcome. These and other forthcoming data will also be published after the patent has been submitted, making reference to this patent. This figure shows an example of an individual ERP (Client) compared to a control group (Control). Here we see that the P300 amplitude (the component at 300 ms.) is increased and exceeds 20 µV. This is a very clear predictor of non-response to Neuromodulation treatment.

Step 3) Prediction of Response to Neuromodulation based on pre-treatment EEG and ERP parameters, any of the observations under a. and b. means that someone will be a responder to neuromodulation treatment, without the presence of any responders as mentioned under steps 1 or 2:
a. Increased frontal fast EEG amplitude (Alpha and/or Beta EEG amplitude). In the preferred embodiment this is the average EEG amplitude in the Alpha frequency band (8-13 Hz) + EEG amplitude in the Beta frequency band (14,5 - 30 Hz) during Eyes Closed at frontal locations (F3, Fz and F4) of above 40 µV, or
b. Increased beta EEG amplitude (14,5 - 30 Hz). In the preferred embodiment this is a beta EEG amplitude during eyes closed of more than 25 µV at Pz, or more than 30 µV at Cz, or more then 20 µV at Fz.
c. No presence of any of the predictors mentioned under step 1 or 2.

An analysis based on 50 patients treated with rTMS showed that in total 65% of the responders to rTMS treatment could be classified with no false positive findings. These and other forthcoming data will also be published after the patent has been submitted, making reference to this patent.

These predictors apply to all patients, and hence the presence of these EEG and ERP profiles will predict treatment outcome with high accuracy and no false positive findings are observed. These predictors might also apply in a similar or reverse order to other treatment modalities such as medication. For example it is expected that a large proportion of responders and especially non-responders to antidepressant medication shows the above EEG and ERP profiles under step 3 and hence will respond to neuromodulation treatment. Using these data patients can hence be offered the right treatment at once.

In figure 2 the algorithm is shown on a real dataset consisting of 50 patients treated with Neuromodulation treatment (rTMS in this case) for depressive complaints. The top 13 clients are non-responders and the bottom 37 clients are responders to this treatment.

The three measures on the left (Oddball P300 amplitude at Pz; EC Slow (Slow EEG activity during Eyes Closed at Pz) and EC APF (Alpha Peak Frequency during Eyes Closed at Fz) are the measures as described under 3: Predictors for Non-Response.

The measure on the left (EC Average Fast EEG (F3, Fz, F4) reflects the average fast EEG amplitude from frontal sites (F3, Fz and F4) for all subjects as described under 2: Predictors for Response.

Also note the 'INVALIDATED' cases' demonstrating that the predictors for non-response have preference over predictors for response and hence invalidates the predictors for response.

Using these cut-off values in this example leads to correct classification of 62% of the non-responders and 65% of the responders with 0% false positives, which is important for the clinical relevance. This example demonstrates the preferred embodiment.

The present invention is not limited to the embodiment shown, but extends also to other embodiments falling within the scope of the appended claims.

## Claims

1. A computer-implemented method for assessing the susceptibility of a human individual suffering from a psychiatric or neurological disorder to neuromodulation treatment, the method comprising:
providing a dataset comprising electroencephalograph (EEG) activity and Event Related Potentials (ERP) data of said human individual;
comparing said dataset with reference data comprising EEG and ERP data from a control group and/or predetermined values;
assessing the susceptibility of said human individual to neuromodulation treatment based on said comparison;
**characterized in that** the susceptibility is assessed as negative if the data in said dataset meets any one of the conditions chosen from a first group of:
a P300 ERP amplitude that is increased compared to the reference data;
an EEG amplitude in the theta and/or the delta frequency range that is increased compared to the reference data;
an alpha peak frequency that is lower than 10Hz.

2. A computer-implemented method according to claim 1, wherein said P300 ERP amplitude comprises a P300 Oddball amplitude.

3. A computer-implemented method according any of the claims 1 or 2, wherein the data comprising the P300 ERP amplitude and/or EEG amplitude in the theta and/or delta frequency range comprises data measured from the Pz location.

4. A computer-implemented method according to any of the preceding claims 1 to 3, wherein the data comprising alpha peak frequency comprises data measured from the Fz location.

5. A computer-implemented method according to any of the preceding claims 1 to 4, wherein the susceptibility is assessed as positive if none of the conditions of the first group are met and
if the data in said dataset meets any one of the conditions chosen from a second group of:
a frontal EEG amplitude in the alpha and/or beta frequency range that is increased compared to the reference data;
an EEG amplitude in the beta frequency range that is increased compared to the reference data.

6. A computer-implemented method according to any of the preceding claims 1 to 5, wherein the data comprising EEG amplitude in the delta and/or the theta frequency range, alpha peak frequency, frontal EEG amplitude in the alpha and/or the beta frequency range and/or the EEG amplitude in the beta frequency range comprises data of the human individual measured with his eyes closed.

7. A computer-implemented method according to any of the preceding claims 1 to 6, wherein the data comprising frontal EEG amplitude in the alpha and/or the beta frequency range comprises data measured from the frontal locations of the scalp of the human individual, preferably from the F3, Fz and/or F4 locations.

8. A computer-implemented method according to any of the preceding claims 1 to 7, wherein the data comprising EEG amplitude in the beta frequency range comprises data measured from one of: the Pz location, the Cz location and the Fz location.

9. Device for use in the method for assessing the susceptibility of a human individual suffering from a psychiatric or neurological disorder to neuromodulation treatment according to any of the preceding claims 1 to 8, wherein the device comprises input means for inputting a dataset comprising EEG and ERP data from the human individual and processing means configured to execute the method according to any of claims 1 to 8 for assessing the susceptibility based on said dataset and output means for outputting said assessment based on the result from the processing means.

## Patentansprüche

1. Rechnergestütztes Verfahren zur Beurteilung des Ansprechens einer Person mit einer psychiatrischen oder neurologischen Erkrankung auf eine neuromodulatorische Behandlung, wobei das Verfahren umfasst:
Vorsehen eines Datensatzes, der elektroenzephalographische (EEG-) Aktivitäts- und ereigniskorrelierte Potentialdaten (ERP) der menschlichen Person umfasst,
Vergleichen des Datensatzes mit Referenzdaten, die EEG- und ERP-Daten aus einer Kontrollgruppe und/oder vorgegebenen Werte umfasst,
Beurteilen des Ansprechens der menschlichen Person auf eine neuromodulatorische Behandlung anhand dieses Vergleichs,
**dadurch gekennzeichnet, dass**
das Ansprechen als negativ gilt, wenn die im Datensatz enthaltenen Daten eine der aus der nachfolgenden ersten Gruppe gewählten Bedingungen erfüllen:
eine P300-ERP-Amplitude, die gegenüber den Referenzdaten erhöht ist,
eine EEG-Amplitude im Theta- und/oder Delta-Frequenzbereich, die gegenüber den Referenzdaten erhöht ist,
eine Alpha-Peak-Frequenz, die unter 10 Hz liegt.

2. Rechnergestütztes Verfahren nach Anspruch 1, wobei die P300-ERP-Amplitude eine P300-Oddball-Amplitude umfasst.

3. Rechnergestütztes Verfahren nach einem der Ansprüche 1 oder 2, wobei die Daten, die die P300-ERP- und/oder EEG-Amplitude im Theta- und/oder Delta-Frequenzbereich umfassen, von der Pz-Stelle aus, gemessene Daten umfassen.

4. Rechnergestütztes Verfahren nach einem der Ansprüche 1 bis 3, wobei die die Alpha-Peak-Frequenz umfassenden Daten Daten, die von der Fz-Stelle aus gemessen wurden, umfassen.

5. Rechnergestütztes Verfahren nach einem der Ansprüche 1 bis 4, wobei das Ansprechen als positiv gilt, wenn keine der Bedingungen der ersten Gruppe erfüllt wird und
wenn die Daten im Datensatz eine der aus der nachfolgenden zweiten Gruppe gewählten Bedingungen erfüllen:
eine frontale EEG-Amplitude im Alpha- und/oder Beta-Frequenzbereich, die gegenüber den Referenzdaten erhöht ist,
eine EEG-Amplitude im Beta-Frequenzbereich, die gegenüber den Referenzdaten erhöht ist.

6. Rechnergestütztes Verfahren nach einem der Ansprüche 1 bis 5, wobei die Daten, die die EEG-Amplitude im Delta- und/oder Theta-Frequenzbereich, Alpha-Peak-Frequenzbereich, die frontale EEG-Amplitude im Alpha- und/oder Beta-Frequenzbereich und/oder die EEG-Amplitude im Beta-Frequenzbereich umfassen, Daten der menschlichen Person umfassen, die bei geschlossenen Augen gemessen wurden.

7. Rechnergestütztes Verfahren nach einem der Ansprüche 1 bis 6, wobei die Daten, die die frontale EEG-Amplitude im Alpha- und/oder Beta-Frequenzbereich umfassen, Daten umfassen, die von den frontalen Stellen der Kopfhaut der menschlichen Person aus, vorzugsweise von den F3-, Fz- und/oder F4-Stellen aus, gemessen wurden.

8. Rechnergestütztes Verfahren nach einem der Ansprüche 1 bis 7, wobei die die EEG-Amplitude im Beta-Frequenzbereich umfassenden Daten Daten, die von einer der nachfolgenden Stellen aus gemessen wurden, umfassen: der Pz-Stelle, der Cz-Stelle und der Fz-Stelle.

9. Vorrichtung zur Verwendung beim Verfahren zur Beurteilung des Ansprechens einer Person mit einer psychiatrischen oder neurologischen Erkrankung auf eine neuromodulatorische Behandlung nach einem der Ansprüche 1 bis 8, wobei die Vorrichtung umfasst: Eingabemittel zum Eingeben eines Datensatzes, der EEG- und ERP-Daten der Person umfassen, und Verarbeitungsmittel, die zum Ausführen des Verfahrens nach einem der Ansprüche 1 bis 8 zur Beurteilung des Ansprechens anhand des Datensatzes konfiguriert sind, sowie Ausgabemittel zum Ausgeben der Beurteilung anhand des Ergebnisses aus den Verarbeitungsmitteln.

## Revendications

1. Procédé mis en oeuvre par ordinateur pour évaluer la susceptibilité d'un être humain qui souffre d'un trouble psychiatrique ou neurologique à un traitement par neuromodulation, le procédé comprenant :
la fourniture d'un jeu de données qui comprend des données d'activité électroencéphalographique (EEG) et de potentiels évoqués cognitifs (ERP) dudit être humain ;
la comparaison dudit jeu de données avec des données de référence qui comprennent des données d'EEG et d'ERP prises parmi un groupe de contrôle et/ou des valeurs prédéterminées ;
l'évaluation de la susceptibilité dudit être humain à un traitement par neuromodulation sur la base de ladite comparaison ;
**caractérisé en ce que** :
la susceptibilité est évaluée comme étant négative si les données dans ledit jeu de données satisfont l'une quelconque des conditions qui est choisie parmi un premier groupe constitué par :
une amplitude d'ERP de l'onde P300 qui est augmentée par comparaison avec les données de référence ;
une amplitude d'EEG dans la plage des fréquences theta et/ou delta qui est augmentée par comparaison avec les données de référence ; et
une fréquence de crête alpha qui est inférieure à 10 Hz.

2. Procédé mis en oeuvre par ordinateur selon la revendication 1, dans lequel ladite amplitude d'ERP de l'onde P300 comprend une amplitude du paradigme oddball de l'onde P300.

3. Procédé mis en oeuvre par ordinateur selon l'une quelconque des revendications 1 ou 2, dans lequel les données qui comprennent l'amplitude d'ERP de l'onde P300 et/ou l'amplitude d'EEG dans la plage des fréquences theta et/ou delta comprennent des données qui sont mesurées à partir de l'emplacement Pz.

4. Procédé mis en oeuvre par ordinateur selon l'une quelconque des revendications qui précèdent 1 à 3, dans lequel les données qui comprennent une fréquence de crête alpha comprennent des données qui sont mesurées à partir de l'emplacement Fz.

5. Procédé mis en oeuvre par ordinateur selon l'une quelconque des revendications qui précèdent 1 à 4, dans lequel :
la susceptibilité est évaluée comme étant positive si aucune des conditions du premier groupe n'est satisfaite ; et
si les données dans le ledit jeu de données satisfont l'une quelconque des conditions qui est choisie parmi un second groupe constitué par :
une amplitude d'EEG frontale dans la plage des fréquences alpha et/ou beta qui est augmentée par comparaison avec les données de référence ;
une amplitude d'EEG dans la plage des fréquences beta qui est augmentée par comparaison avec les données de référence.

6. Procédé mis en oeuvre par ordinateur selon l'une quelconque des revendications qui précèdent 1 à 5, dans lequel les données qui comprennent une amplitude d'EEG dans la plage des fréquences delta et/ou theta, une fréquence de crête alpha, une amplitude d'EEG frontale dans la plage des fréquences alpha et/ou beta et/ou l'amplitude d'EEG dans la plage des fréquences beta comprennent des données de l'être humain qui sont mesurées tandis que ses yeux sont fermés.

7. Procédé mis en oeuvre par ordinateur selon l'une quelconque des revendications qui précèdent 1 à 6, dans lequel les données qui comprennent une amplitude d'EEG frontale dans la plage des fréquences alpha et/ou beta comprennent des données qui sont mesurées à partir d'emplacements frontales du cuir chevelu de l'être humain, de préférence à partir d'emplacements F3, Fz et/ou F4.

8. Procédé mis en oeuvre par ordinateur selon l'une quelconque des revendications qui précèdent 1 à 7, dans lequel les données qui comprennent une amplitude d'EEG dans la plage des fréquences beta comprennent des données qui sont mesurées à partir d'un parmi : l'emplacement Pz, l'emplacement Cz et l'emplacement Fz.

9. Dispositif destiné à être utilisé au niveau du procédé permettant d'évaluer la susceptibilité d'un être humain qui souffre d'un trouble psychiatrique ou neurologique à un traitement par neuromodulation selon l'une quelconque des revendications qui précèdent 1 à 8, dans lequel le dispositif comprend des moyens d'entrée pour entrer un jeu de données qui comprend des données d'EEG et d'ERP de l'être humain et des moyens de traitement qui sont configurés de manière à ce qu'ils exécutent le procédé selon l'une quelconque des revendications 1 à 8 pour évaluer la susceptibilité sur la base dudit jeu de données et des moyens d'émission en sortie pour émettre en sortie ladite évaluation sur la base du résultat en provenance du moyen de traitement.
